# EUROPEAN PATENT APPLICATION

(11) **EP 4 292 601 A1**
(43) Date of publication of application: **20.12.2023**
(21) Application number: 21925943.9
(22) Date of filing: 29.12.2021
(51) Int. Cl.: A61K 38/12, A61K 38/05, A61P 19/10, A61P 21/00, A23L 33/18, A23K 20/147

(54) **COMPOSITION FOR TREATING SARCOPENIA OR OSTEOPOROSIS THROUGH MECHANISM PROMOTING FORMATION OF MUSCLE FIBERS OR INHIBITING OSTEOCLASTOGENESIS, COMPRISING CYCLO-L-PHENYLALANYL-L-PROLINE DIPEPTIDES**

(30) Priority: 09.02.2021 KR 20210018445
(71) Applicant: Cellbion Co., Ltd., Seoul 03080 (KR)
(72) Inventor: KIM, Hong Hee, Seongnam-si Gyeonggi-do 13646 (KR); KWON, Jun Oh, Seoul 03100 (KR); JUNG, Su Han, Seoul 02844 (KR); HONG, Seo Jin, Goyang-si Gyeonggi-do 10384 (KR)
(74) Representative: Bates, Philip Ian
(86) International application number: PCT/KR2021/020154
(87) International publication number: WO 2022/173123

(57) **Abstract**

The present invention relates to a composition for preventing or treating sarcopenia or osteoporosis, comprising cyclo-L-phenylalanyl-L-proline dipeptides. The composition for preventing or treating sarcopenia or osteoporosis, comprising cyclo-L-phenylalanyl-L-proline dipeptides, according to the present invention, inhibits osteoclastogenesis, which is associated with a decrease in bone density, and promotes the formation of muscle fibers, so as to increase muscle mass, and thus can prevent or treat osteoporosis.

## Description

### BACKGROUND OF THE INVENTION

### 1. Field of the Invention

The present invention relates to a composition for treating osteoporosis due to muscle loss and decreased bone density, more specifically to a composition for treating osteoporosis through the mechanism of promoting muscle fiber formation or inhibiting osteoclastogenesis, comprising cyclo-L-phenylalanyl-L-proline dipeptides.

### 2. Description of the Related Art

As the body ages, fat in the body increases, solids and moisture decrease, and bone mass decreases due to the loss of calcium, especially in the bones, resulting in lower bone density. In addition, the cartilage tissue becomes thinner and the elasticity weakens, causing arthritis, and the volume of the voluntary muscle decreases, resulting in poor motor ability.

Among the structural changes of the body, in particular, decreased motor ability and decreased bone density become factors that not only make daily life inconvenient in old age but also significantly increase the risk of fracture.

Hip fracture refers to a fracture around the hip joint, and it mainly occurs in the elderly over 70 years of age with severe osteoporosis. The main cause of hip fracture is a fall, and there are various causes that contribute to this, but the weakening of muscle strength and the decrease in bone density can be cited as the main causes.

Osteoporosis and senile sarcopenia are increasingly interested with the aging of the population, but no significant solution has been found yet. Specifically, hormones, bisphosphonates, RANKL antibodies, and PTH peptides are used as treatments for osteoporosis, but there are restrictions on the subject and duration of use, and side effects have been reported. In addition, it has been reported that there is a clinical need for drugs with a good feeling of use because conventional drugs have low compliance at the time of taking. In order to increase muscle volume, biologic drugs are being developed targeting myostatin and activin receptors that reduce muscle mass, but relatively low efficacy and side effects have been reported.

Cyclic di-peptide (CDP) is a type of dipeptide made by a peptide bond between two α-amino acids. Compared to a general dipeptide made of a single peptide bond, cyclic dipeptides have two peptide bonds to form a ring structure.

These small molecules are substances produced by physiochemical reactions or obtained as by-products in the process of microbial fermentation. There are various types of cyclic dipeptides depending on the constituent amino acids, but there are common characteristics. The peptide bonds give stability to physical and chemical reactions, while the ring structure makes them hydrophobic, which can facilitate intracellular penetration.

In order to understand the physiological effects of cFP on mammals, the present inventor tried to investigate the effect of oral administration of cFP on the musculoskeletal system of a mouse. As a result, the present inventors have confirmed novel uses of the cyclo-L-phenylalanyl-L-proline dipeptides related to the prevention or treatment of sarcopenia and osteoporosis, leading to completion of the present invention.

### SUMMARY OF THE INVENTION

An object of the composition for treating osteoporosis according to one embodiment of the present invention is to prevent or treat osteoporosis by inhibiting osteoclastogenesis associated with a decrease in bone density.

An object of the composition for treating osteoporosis according to another embodiment of the present invention is to prevent or treat sarcopenia by promoting the formation of muscle fibers.

The objects of the present invention are not limited to those mentioned above, and other objects not mentioned can be clearly understood by those skilled in the art from the following description.

To achieve the above objects, in one aspect of the present invention, the present invention provides a composition for preventing or treating sarcopenia or osteoporosis comprising cyclo-L-phenylalanyl-L-proline dipeptides represented by the following formula.

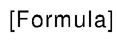

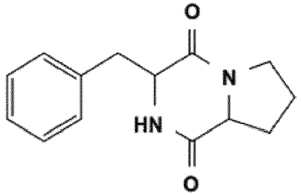

Herein, the therapeutic composition may further comprise a pharmaceutically acceptable carrier, an excipient, or a diluent.

In another aspect of the present invention, the present invention provides a composition for promoting the differentiation of myoblasts and/or inhibiting the formation of osteoclasts comprising cyclo-L-phenylalanyl-L-proline dipeptides represented by the following formula.

In another aspect of the present invention, the present invention provides a quasi-drug composition comprising cyclo-L-phenylalanyl-L-proline dipeptides.

In another aspect of the present invention, the present invention provides a health functional food composition for muscle increasing and/or bone strengthening comprising cyclo-L-phenylalanyl-L-proline dipeptides.

In another aspect of the present invention, the present invention provides a feed additive composition for muscle increasing and/or bone strengthening in edible livestock comprising cyclo-L-phenylalanyl-L-proline dipeptides.

In another aspect of the present invention, the present invention provides a method for preventing or treating sarcopenia or osteoporosis, containing a step of administering the composition comprising cyclo-L-phenylalanyl-L-proline dipeptides to a non-human subject.

In another aspect of the present invention, the present invention provides a composition for promoting the formation of muscle fibers comprising cyclo-L-phenylalanyl-L-proline dipeptides represented by the following formula.

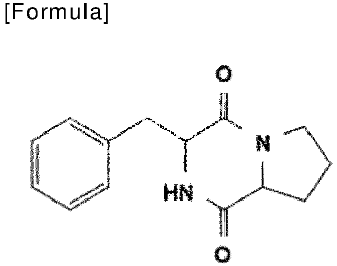

In another aspect of the present invention, the present invention provides a composition for inhibiting the differentiation of osteoclasts comprising cyclo-L-phenylalanyl-L-proline dipeptides represented by the following formula.

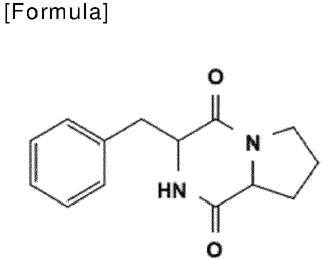

In the present invention, the term "prevention" refers to any act of inhibiting or delaying the onset of sarcopenia or osteoporosis by administering the composition according to the present invention.

In the present invention, the term "treatment" refers to any act of inhibiting, reducing or alleviating the symptoms of sarcopenia or osteophorosis by administering the composition according to the present invention.

The pharmaceutical composition of the present invention can be used as a single preparation, or can be prepared and used as a combined preparation by further including a drug known to have a therapeutic effect on sarcopenia or osteoporosis.

The pharmaceutical composition of the present invention may further comprise a pharmaceutically acceptable carrier.

The above "pharmaceutically acceptable" means that it does not significantly stimulate living organisms and does not inhibit the biological activity and properties of the administered active substance.

The carrier may be a natural or non-natural carrier. Depending on the formulation, the composition of the present invention may be formulated using various carriers such as diluents or excipients such as fillers, extenders, binders, wetting agents, disintegrants, and surfactants. For example, solid formulations for oral administration include tablets, pills, powders, granules and capsules. These solid formulations are prepared by mixing one or more compounds of the present invention with one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized preparations and suppositories. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The pharmaceutical composition can be any one formulation selected from the group consisting of tablets, pills, powders, granules, capsules, suspensions, solutions, emulsions, syrups, sterilized aqueous solutions, water-insoluble excipients, suspensions, emulsions, lyophilized preparations, and suppositories.

In addition, the pharmaceutical composition of the present invention can contain a pharmaceutically effective amount of cyclo-L-phenylalanyl-L-proline dipeptides.

The term "pharmaceutically effective amount" means an amount sufficient to treat a disease with a reasonable benefit/risk ratio applicable to medical treatment. The effective dose level depends on the factors including subject type and severity, age, gender, drug activity, sensitivity to drug, time of administration, administration route and excretion rate, duration of treatment, concomitant drugs, and other factors well known in the medical field.

The pharmaceutical composition of the present invention can be administered alone or in combination with other therapeutic agents. In combination administration, the administration can be sequential or simultaneous. In addition, the pharmaceutical composition of the present invention can be administered in single or multiple doses.

It is important to administer the amount that can obtain the maximum effect with the minimum amount without causing side effects in consideration of all the above factors, and such dosage can be easily determined by those skilled in the art.

Preferably, the pharmaceutical composition according to the invention may contain cyclo-L-phenylalanyl-L-proline dipeptides in an amount of 0.001 to 1500 *µ*g/ml, more preferably in an amount of 0.001 to 1000 *µ*g/ml.

In another aspect of the present invention, the present invention provides a health functional food composition for preventing or ameliorating sarcopenia or osteoporosis comprising cyclo-L-phenylalanyl-L-proline dipeptides.

In the present invention, the term "amelioration" refers to any act that improves or benefits the symptoms of individuals suspected of or suffering from sarcopenia or osteoporosis by using the composition.

The food composition of the present invention may further include a sitologically acceptable carrier.

The food composition may have a function to help suppress sarcopenia or osteoporosis.

The food composition of the present invention includes forms, such as pills, powders, granules, precipitates, tablets, capsules, or liquids. The food herein is not particularly limited. For example, the cyclo-L-phenylalanyl-L-proline dipeptides of the present invention can be added to various beverages, gums, teas, vitamin complexes, health supplements, etc.

In addition to the cyclo-L-phenylalanyl-L-proline dipeptides, other ingredients that do not interfere with the sarcopenia or osteoporosis inhibitory activity can be added to the food composition, and the type thereof is not particularly limited. For example, like conventional foods, the food composition of the present invention can contain additional ingredients such as various herbal extracts, sitologically acceptable food supplement additives, or natural carbohydrates.

The term "food supplement additive" used in this invention refers to a component that can be added to food supplementally, and can be appropriately selected and used by those skilled in the art as being added to produce a health functional food of each formulation. Examples of the food supplement additives include various nutrients, vitamins, minerals (electrolytes), flavors including natural flavors and synthetic flavors, coloring agents and fillers, pectic acid and its salts, alginic acid and its salts, organic acids, protective colloidal viscosifiers, pH regulators, stabilizers, antiseptics, glycerin, alcohol, carbonators used in carbonated beverages, etc., but the types of the food supplement additives of the present invention are not limited by the above examples.

The natural carbohydrates above can be one of monosaccharides such as glucose and fructose; disaccharides such as maltose and sucrose; polysaccharides such as dextrin and cyclodextrin; and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent.

The food composition can be used for the preparation of health functional foods or can be included in health functional foods.

The term "health functional food" used in this invention refers to food prepared and processed in the form of tablets, capsules, powders, granules, liquids and pills using raw materials or ingredients having useful functionality for the human body. Herein, the functionality means obtaining useful effects for health applications such as controlling nutrients or physiological functions on the structure and function of the human body. The health functional food of the present invention can be prepared by a method commonly used in the art, and can be prepared by adding raw materials and ingredients commonly used in the art. Unlike general drugs, the health functional food of the present invention also has the advantage of not causing side effects that may occur when taking drugs for a long time because it is based on food as a raw material, and can be excellent in portability.

The composition of the present invention can be included in a health functional food. In that case, the composition can be added as it is or as mixed with other food components according to the conventional method. The mixing ratio of active ingredients can be regulated according to the purpose of use (prevention, health or treatment).

In general, to produce food, the cyclo-L-phenylalanyl-L-proline dipeptides of the present invention can be added in an amount of 0.1 to 1 weight%, preferably 0.5 to 1 weight% of the raw material composition. However, if long term administration is required for health and hygiene or regulating health condition, the content can be lower than the above.

The food herein is not limited. For example, the composition of the present invention can be added to meats, sausages, breads, chocolates, candies, snacks, cookies, pizza, ramyuns, flour products, gums, dairy products including ice cream, soups, beverages, teas, drinks, alcohol drinks and vitamin complexes, etc, and in wide sense, almost every food applicable in the production of health functional food can be included.

There is no particular limitation on the type of health functional food that may contain the composition according to an embodiment of the present invention. Specific examples include meats, sausages, breads, chocolates, candies, snacks, cookies, pizza, ramyuns, flour products, gums, dairy products including ice cream, soups, beverages, teas, drinks, alcohol drinks and vitamin complexes, etc, and may include all health functional foods, and may include foods used as feed for animals.

In addition, when the health functional food composition of the present invention is used in the form of a beverage, it may contain various sweeteners, flavoring agents, or natural carbohydrates as additional ingredients, like conventional beverages. The natural carbohydrates above can be one of monosaccharides such as glucose and fructose, disaccharides such as maltose and sucrose, polysaccharides such as dextrin and cyclodextrin, and glucose alcohols such as xilytole, sorbitol and erythritol. The content of the natural carbohydrate can be preferably about 0.01 to 0.04 g and more preferably 0.02 to 0.03 g per 100 mℓ of the composition according to an embodiment of the present invention, but not always limited thereto. The sweetener can be a natural sweetener such as thaumatin and stevia extract, and a synthetic sweetener such as saccharin and aspartame.

In addition to the ingredients mentioned above, the health functional food composition of the present invention can include in a variety of nutrients, vitamins, minerals, flavors, coloring agents, pectic acid and its salts, alginic acid and its salts, organic acids, protective colloidal viscosifiers, pH regulators, stabilizers, antiseptics, glycerin, alcohol, carbonators which used to be added to soda, etc. The composition of the present invention can also include natural fruit juice, fruit beverages and/or fruit flesh addable to vegetable beverages.

In another aspect of the present invention, the present invention provides a method for preventing or treating sarcopenia or osteoporosis, comprising a step of administering cyclo-L-phenylalanyl-L-proline dipeptides to a subject at risk of developing sarcopenia or osteoporosis or suffering from sarcopenia or osteoporosis.

In the present invention, the subject refers to all animals including humans that have or may develop sarcopenia or osteoporosis. By administering a pharmaceutical composition comprising a compound of the present invention or a pharmaceutically acceptable salt thereof to a subject suspected of having sarcopenia or osteophorosis, the subject can be treated efficiently.

Specifically, the subject is a subject in need of prevention or treatment of sarcopenia or osteoporosis and may be humans as well as mammals such as cows, horses, sheep, pigs, goats, camels, nutrition, dogs, and cats in need of treatment of similar symptom, but not always limited thereto.

The term "administration" used in the present invention means introducing the pharmaceutical composition of the present invention to a subject by any suitable method. The composition according to an embodiment of the present invention can be administered through various routes, either oral or parenteral, as long as it can reach the target tissue.

The effective amount of a compound of the present invention or a pharmaceutically acceptable salt thereof indicates a pharmaceutically effective amount.

The term "pharmaceutically effective dose" used in the present invention means an amount sufficient to treat a disease with a reasonable benefit/risk ratio applicable to medical treatment or improvement. The effective dose level depends on the factors including subject type and severity, age, gender, type of disease, drug activity, sensitivity to drug, time of administration, administration route and excretion rate, duration of treatment, concomitant drugs, and other factors well known in the medical field.

The composition of the present invention can be administered alone or in combination with other therapeutic agents. In combination administration, the administration can be sequential or simultaneous. In addition, the pharmaceutical composition of the present invention can be administered in single or multiple doses. It is important to administer the amount that can obtain the maximum effect with the minimum amount without causing side effects in consideration of all the above factors, and such dosage can be easily determined by those skilled in the art.

The preferred dosage of the composition of the present invention can be determined according to weight and condition of a patient, severity of a disease, preparation of a drug, administration pathway and time. A suitable total daily usage can be determined by a doctor within the scope of medical judgment, but is generally 0.001 to 1000 mg/kg, preferably 0.05 to 200 mg/kg, and more preferably 0.1 to 100 mg/kg. The administration frequency can be once a day or a few times a day.

The method of administration includes without limitation as long as it is a conventional method in the art. For example, the composition can be administered by oral, rectal, intravenous, intramuscular, subcutaneous, intrauterine, intrathecal or intracerebrovascular injection.

### ADVANTAGEOUS EFFECT

The composition for treating osteoporosis according to one embodiment of the present invention can prevent or treat osteoporosis by inhibiting osteoclastogenesis associated with a decrease in bone density.

The composition for treating osteoporosis according to another embodiment of the present invention can prevent or treat sarcopenia by promoting the formation of muscle fibers.

The effects of the present invention are not limited to those mentioned above, and other effects not mentioned can be clearly understood by those skilled in the art from the following description.

### BRIEF DESCRIPTION OF THE DRAWINGS

Figure 1 is a set of photographs and graphs showing the results of promoting muscle fiber formation of myoblasts according to cFP treatment ((A) myotubes stained by LADD. Scale bar = 100 µm.; (B) The diameter of myotubes and the number of nuclei in myotubes depending on the concentration of cFP treatment and vehicle).
Figure 2 is a set of photographs and a graph showing the results of inhibiting osteoclastogenesis according to cFP treatment ((A) osteoclasts stained by TRAP and (B) the number of osteoclasts depending on the concentration of cFP treatment and vehicle).
Figure 3 is a set of graphs showing the increase in muscle mass in mice according to oral administration of cFP by gavage feeding.
Figure 4 is a set of graphs showing the effects on the trabecular bone of mouse femur in the case of free drinking of cFP.

### DESCRIPTION OF THE PREFERRED EMBODIMENTS

The objects and effects of the present invention, and the technical configurations for achieving them, will become clear with reference to the embodiments described below in detail together with the accompanying drawings. In describing the present invention, if it is determined that a detailed description of a known function or configuration may unnecessarily obscure the gist of the present invention, the detailed description will be omitted. And the terms described later may vary according to the intention of the user and the operator or custom.

However, the present invention is not limited to the embodiments disclosed below and may be implemented in a variety of different forms. These embodiments are provided only to make the disclosure of the present invention complete and to fully inform the scope of the invention to those skilled in the art, and this invention is only defined by the scope of the claims. Therefore, the definition should be made based on the contents throughout this specification.

Muscle growth is due to the formation of muscle fibers according to the binding between individual myoblasts. Therefore, the improvement of muscle growth can be estimated through the degree of muscle fiber formation in the environment that induced the binding of myoblasts. Accordingly, myoblasts were isolated from the muscle tissue of a subject and cultured, and whether muscle fibers were formed using the isolated myoblasts was observed.

The formation of muscle fibers can be evaluated by measuring the number of myoblasts that form muscle fibers and the thickness of the formed muscle fibers. To this end, a high-magnification optical microscope was used to identify the nucleus in the muscle fiber through muscle fiber staining. The thickness of the muscle fiber was also measured.

The differentiation of osteoclasts occurs by intercellular binding as the differentiation of individual bone marrow-derived macrophages proceeds. Accordingly, only bone marrow-derived macrophages were isolated and cultured, and the separated macrophages were differentiated into osteoclasts.

In order to determine the degree of differentiation of the differentiated osteoclasts, TRAP (tartrate-resistant acid phosphatase) stain assay was used. When macrophages differentiate into osteoclasts, they produce TRAP, which can be stained to quantify the degree of osteoclast differentiation. In addition, in order to confirm the maturity of osteoclasts, the number of cells constituting osteoclasts was identified. As described above, the degree of differentiation of osteoclasts was analyzed using two indicators: the number of nuclei in osteoclast and TRAP (tartrate-resistant acid phosphatase) stain.

According to an embodiment of the present invention, the increase in muscle weight and bone mass by oral administration of cyclo-L-phenylalanyl-L-proline (cFP) was measured. Muscle weight was measured by separating the muscles of the mouse according to anatomical muscle classification. In addition, trabecular bone analysis on the femur of the mouse was performed using high-resolution micro-CT (SMX-90CT system). Based on the growth plate of the femur, the lower part of the 1mm was the starting point of the analysis, and the analysis was conducted from the starting point to 1.5 mm below the bone. Each indicator means as follows. bone volume/total bone volume (BV/TV, %), bone surface/trabecular bone volume (BS/BV, 1/mm), trabecular thickness (Tb.Th, mm), trabecular number (Tb.N, 1/mm), trabecular separation (Tb.Sp, mm).

Hereinafter, the present invention will be described in detail by the following examples.

### Mode for Embodying the invention

### Example 1: Differentiation of mvoblasts according to cFP treatment

### <1-1> Preparation of mvoblasts

After sacrificing the 5-week-old male CrljOri : CD1 (ICR) mice, the hind limb muscle tissues were obtained. The muscle tissue was minced with a scalpel for 5 minutes. The minced muscle solution was incubated at 37°C for 60 minutes and then centrifuged at 1500 rpm for 5 minutes. The obtained cells were suspended in Ham's F-10 medium containing 20% heat-inactivated horse serum and 50 units/ml of penicillin in the presence of basic FGF (2.5 ng/ml), and then plated on a cell culture dish for 1 hour without gelatin coating. After 1 hour, the suspended cells were transferred to a secondary cell culture dish without gelatin coating and cultured for 1 hour. After repeating this process, the cells were transferred to a gelatin-coated dish. The cultured cells were used as myoblasts.

### <1-2> Differentiation of mvoblasts and LADD staining (muscle fiber formation experiment)

Myoblasts obtained from the muscle tissue were plated on a 48-well tissue culture plate pretreated with 10% Matrigel at the density of 3 × 10⁴ cells per well, followed by culture. After culturing for about 24 hours, the cells were further cultured in a DEM fusion medium containing 5% heat-inactivated horse serum and 50 units/ml of penicillin/streptomycin. The medium was replaced once every 2 days. After culturing, the cells on the 48-well plate were washed with PBS (phosphate buffered saline) and fixed using 70% ethanol. Ethanol was removed, and the formed muscle fibers were stained using LADD staining reagent including toluidine blue and fuchsin. After incubation for 1 minute, the stained cells were washed with distilled water until the LADD staining solution was not leached into water. After washing, the cells were dried at room temperature for 10 minutes.

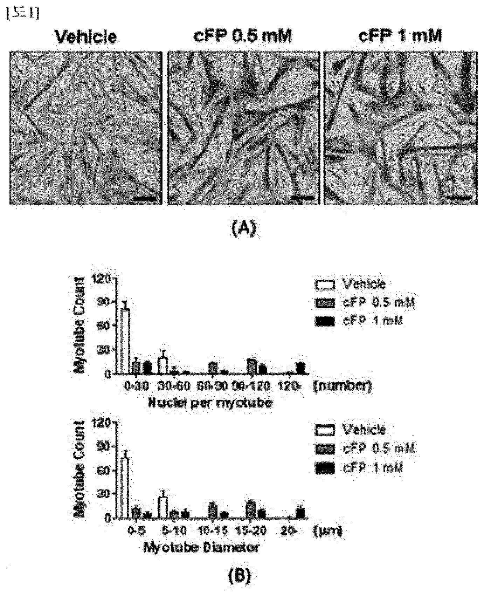

Figure 1 is a set of photographs and graphs showing the results of promoting muscle fiber formation of myoblasts according to cFP treatment. As shown in Figure 1, it was confirmed that the culture medium of myoblasts treated with cFP promoted the fusion of myoblasts compared to the culture medium of myoblasts treated with vehicle ((A) Myotubes stained with LADD staining. Scale bar = 100 µm.; (B) Diameter of myotube and number of nuclei of myotube according to cFP treatment by vehicle and concentration. ANOVA-Tukey test was performed.)

### Example 2: Osteoclast formation according to cFP treatment

### <2-1> µCT analysis

The mouse femur was analyzed with high resolution µCT (SMX-90CT system; Shimadzu, Kyoto, Japan). The scanning images of µCT were reconstructed by the VG Studio MAX 1.2.1 program (Volume Graphics, Heidelberg, Germany). Each three-dimensional image was analyzed using TRI/3D-VIE (RATOC System Engineering, Kyoto, Japan) to measure the bone volume, cortical bone volume, trabecular number, and trabecular separation. The 3D images were obtained from the CT Volume program (Version 1.11, SkyScan).

### <2-2> Preparation of bone marrow-derived macrophages (BMM)

After sacrificing the 5-week-old male CrljOri : CD1 (ICR) mice, the femurs and tibias were obtained from the mice. Bone marrow cells were obtained by washing the bone marrow from the femur and tibia. Non-adherent bone marrow cells were further cultured in α-MEM containing M-CSF (30 ng/ml) for 3 days to generate bone marrow-derived macrophages (BMM).

### <2-3> Osteoclast differentiation and tartrate-resistant acid phosphatase (TRAP) staining

To generate osteoclasts, BMMs were cultured in a 48-well plate (4 × 10⁴ cells/well) for 4 days with α-MEM complete medium containing 10% (v/v) heat-inactivated fetal bovine serum (FBS) and 50 units/ml of penicillin/streptomycin in the presence of M-CSF (30 ng/ml) and RANKL (100 ng/ml). The medium was replaced every 3 days. Multinuclear cells (MNC) were observed on day 4. The cells were fixed in 10% formalin solution for 20 minutes and then permeabilized with 0.1% Triton X-100 for 1 minute. After washing twice with PBS, the cells were stained using Leukocyte Acid Phosphatase Assay Kit (Sigma-Aldrich) according to the manufacturer's instructions. The degree of differentiation was evaluated by determining the number of differentiated osteoclasts per well based on osteoclasts having 5 or more nuclei in the cell.

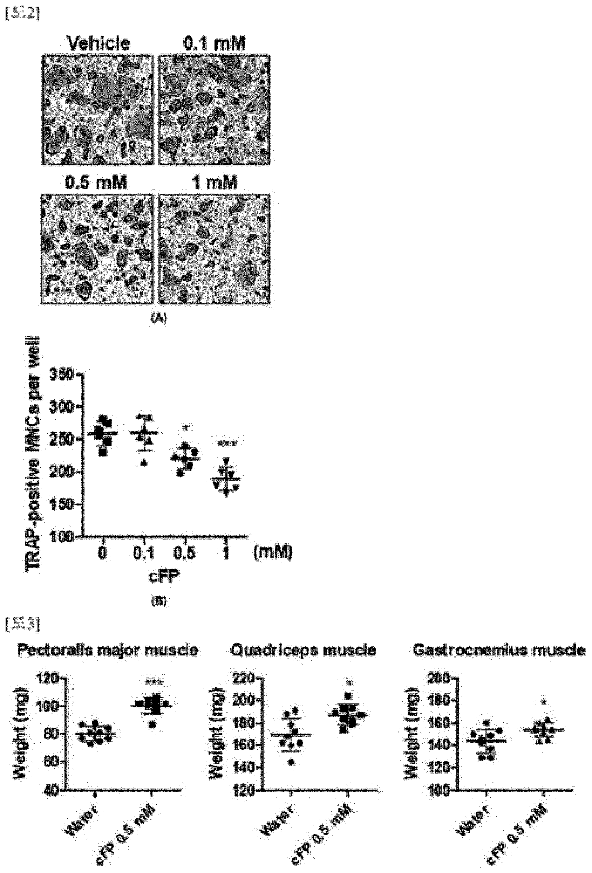

Figure 2 is a set of photographs and a graph showing the results of inhibiting osteoclastogenesis according to cFP treatment concentration. As shown in Figure 2, it was confirmed that the differentiation of bone marrow-derived macrophages into osteoclasts was inhibited in a concentration-dependent manner by cFP.

### Example 3: Analysis of muscle and trabecular bone after oral intake of cFP

### <3-1> Preparation of experimental mice

Animal studies were conducted in accordance with the guidelines for animal research. All experimental procedures were approved by the Animal Care and Use Committee of Seoul National University Animal Hospital. Male C57BL/6J mice at 4 or 6 weeks of age were purchased from Orient Bio Inc. (Seongnam, Korea) and housed in a specific pathogen-free (SPF) animal facility with controlled temperature (22°C), humidity (55%), and a 12-hour light-dark cycle. All animals were provided with a free feeding and drinking system. Oral gavage was performed every 24 hours by administering cFP through a stainless steel supply tube, and 100 ul of the solution was orally administered at a time of administration. Alternatively, cFP was mixed with drinking water and administered through a free drinking system. The mice were sacrificed in a carbon dioxide euthanasia chamber.

### <3-2> Muscle collection and weighing

After euthanizing the mice using CO₂ gas, the pectoralis major muscle, quadriceps muscle, and gastrocnemius muscle were separated according to the anatomical classification of the muscle. The separated muscles were weighed using a microbalance.

### <3-3> Analysis of trabecular bone of femur

Using a micro-CT machine, overall analysis data on the femur separated from the euthanized mice were obtained. Among the data obtained, 1 mm in the distal direction of the growth plate of the mouse was set as the starting point, and 1.5 mm in the distal direction was designated as a range. Then, the trabecular bone portion was remodeled and analyzed to obtain indicators of the trabecular bone.

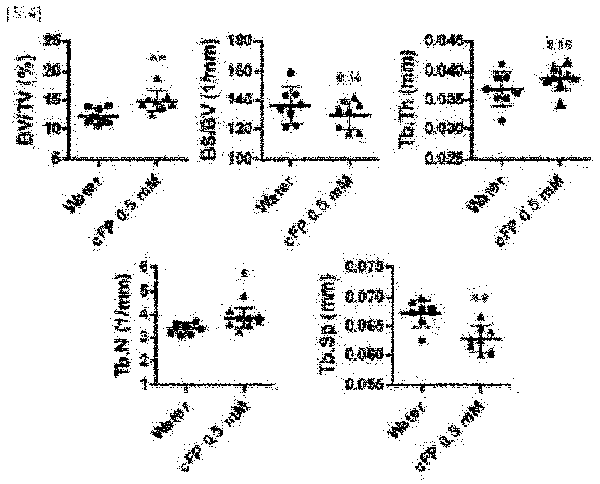

Figure 3 is a set of graphs showing the increase in muscle mass in mice according to oral administration of cFP by gavage feeding. Figure 4 is a set of graphs showing the effects on the trabecular bone of mouse femur in the case of free drinking of cFP.

As shown in Figure 3, it was confirmed that the muscle mass of the mice was increased by oral administration of cFP by gavage compared to the control group. As shown in Figure 4, it was confirmed that the trabecular bone indices of BV/TV (bone volume/total bone volume), Tb.Th (trabecular thickness), and Tb.N (trabecular number) in the cFP-administered group were significantly high, while the trabecular bone indices of BS/BV (bone surface/trabecular bone volume), TB.Pf (trabecular bone pattern factor), and Tb.Sp (trabecular separation) in the purified water-administered group were low.

In summary of the above examples, it was confirmed that cyclo-L-phenylalanyl-L-proline dipeptides can increase the amount of muscle in the body and inhibit osteoclastogenesis through oral administration in relation to muscle loss and osteoclastogenesis that cause sarcopenia or osteoporosis.

In this specification and drawings, preferred embodiments of the present invention are disclosed, and although specific terms are used, they are only used in a general sense to easily explain the technical content of the present invention and help understanding of the present invention, and are not intended to limit the scope of the present invention. It is obvious to those skilled in the art that other modifications based on the technical idea of the present invention can be implemented in addition to the embodiments disclosed herein.

### Industrial Applicability

The present invention can be applicated to the prevention or treatment of sarcopenia or osteoporosis.

## Claims

1. A composition for preventing or treating sarcopenia or osteoporosis, comprising cyclo-L-phenylalanyl-L-proline dipeptides of the following formula.

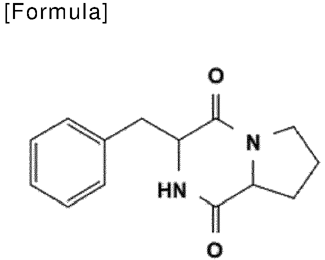

2. The composition according to claim 1, wherein the composition further comprises a pharmaceutically acceptable carrier, an excipient, or a diluent.

3. A quasi-drug composition for preventing or improving sarcopenia or osteoporosis, comprising cyclo-L-phenylalanyl-L-proline dipeptides of the following formula.

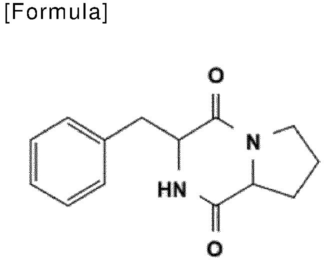

4. A health functional food composition for muscle increasing and/or bone strengthening, comprising cyclo-L-phenylalanyl-L-proline dipeptides of the following formula.

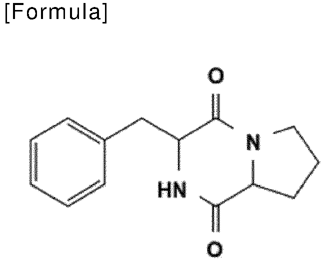

5. A feed additive composition for muscle increasing and/or bone strengthening in edible livestock, comprising cyclo-L-phenylalanyl-L-proline dipeptides of the following formula.

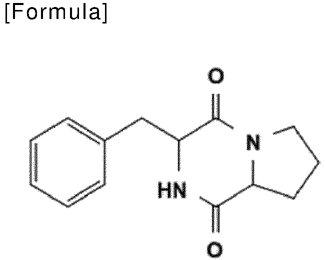

6. A method for treating sarcopenia or osteoporosis, comprising a step of administering an effective amount of cyclo-L-phenylalanyl-L-proline dipeptides of the following formula to a subject in need thereof.

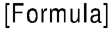

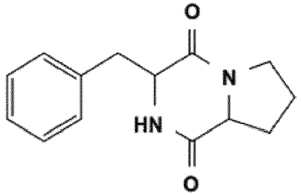

7. The use of cyclo-L-phenylalanyl-L-proline dipeptides of the following formula for producing a drug for treating of sarcopenia or osteoporosis.
